# EUROPEAN PATENT APPLICATION

(11) **EP 4 563 710 A1**
(43) Date of publication of application: **04.06.2025**
(21) Application number: 23847018.1
(22) Date of filing: 27.07.2023
(51) Int. Cl.: C12Q 1/6869, C12Q 1/6827

(54) **METHOD FOR VERIFYING NEXT-GENERATION SEQUENCING PANELS**

(30) Priority: 28.07.2022 KR 20220093705
(71) Applicant: National Cancer Center, Goyang-si, Gyeonggi-do 10408 (KR)
(72) Inventor: HONG, Kyeong-Man, Paju-si, Gyeonggi-do 10895 (KR); KIM, Young-Ho, Paju-si, Gyeonggi-do 10871 (KR)
(74) Representative: J A Kemp LLP
(86) International application number: PCT/KR2023/010929
(87) International publication number: WO 2024/025361

(57) **Abstract**

The present invention relates to: a composition for validating next generation sequencing (NGS) panels, comprising homozygote DNA and control genomic DNA; a kit for validation of NGS panels, comprising the composition; a validation method for NGS panels through the analysis of false negative variants, limit of detection, and false positive variants; and a method for providing information to enhance the specificity of NGS panels. In particular, the validation method of the present invention enables the objective analysis of the frequency of false negative variants, the frequency of false positive variants, and the limit of detection for NGS panels, making it effectively usable in the validation of NGS panels.

## Description

### TECHNICAL FIELD

The present invention relates to a composition for validating next generation sequencing panels, a kit for validating next generation sequencing panels that includes the composition, and a validation method for next generation sequencing panels.

### BACKGROUND

Next generation sequencing (hereinafter referred to as "NGS") is a technology that enables the simultaneous analysis of millions of nucleotide sequences, also known as massively parallel sequencing or high-throughput sequencing. In contrast to Sanger sequencing, which previously cost billions of dollars to analyze an individual's genome, the NGS method allows for genome analysis at a cost of about $1,000 over several days. Currently, NGS technology is being applied in various fields, including the screening, diagnosis, prognosis prediction, and selection of therapeutic agents for genetic diseases, infectious diseases, and cancer. In the United States alone, over 55,000 NGS diagnostic panels utilizing this technology are employed in clinical practice for more than 11,000 diseases.

In order to apply the developed NGS panel to clinical practices it is essential to evaluate whether the panel can accurately produce the desired results. Accordingly, the U.S. FDA has issued guidelines for the validation of NGS panels, while the Center for Medicare & Medicaid Services (CMS) monitors these panels. Both guidelines and monitoring methods are continuously updated to ensure their effectiveness.

Currently, the most widely used method for validating NGS panels is the approach introduced by Frampton et al. in Nature Biotechnology in 2013 (hereinafter referred to as the "Frampton method"), which has been primarily adopted in subsequent studies (Shin et al., Nature Comm 2017; and Froyen et al., Cancers 2022). The Frampton method involves validating NGS panels using a DNA pool mixed with DNA from HapMap cell lines, whose nucleotide sequences are well characterized, or a DNA pool mixed with DNA from cancer cell lines. Specifically, the Frampton method combines equal amounts of DNA from 10 HapMap cell lines to create a DNA mixture with 5% DNA, referred to as pool XX, and similarly prepares another mixture from a different set of 10 cell lines, called pool YY, which is then analyzed using NGS panels to evaluate sensitivity and specificity (Frampton et al., Nature Biotechnology 2013). The proportion of each single nucleotide variant (SNV) allele in a DNA mixture (hereinafter referred to as "variant allelic fraction" or VAF) can be calculated. A haplotype allele present in only one cell line will account for 5% of the entire mixture, while remaining SNVs appearing in multiple instances will have a VAF of 10% or more. When analyzing the panel using a mixture of HapMap cell lines selected by Frampton et al., it was found that 20% of the alleles included in the panel's genes exhibited a VAF of about 5%, and approximately 30% of the alleles displayed a VAF of 10%. Therefore, 20% of the alleles could be utilized to measure the detection rate of 5% VAF, while 30% of the alleles could assess the detection rate of 10% VAF. However, in Frampton et al.'s study, the HapMap cell line mixture created did not contain alleles with a VAF of less than 5%, meaning that the detection rate for alleles with a VAF below 5% could not be measured. Moreover, to determine the detection rate of alleles with a 1% VAF using the Frampton method, at least 50 HapMap cell lines would be necessary, and variants with a discovery frequency around 1% must be included. Additionally, Frampton's results suggested that while approximately 200 alleles with a VAF of about 5% could be identified using 10 HapMap cell lines, it could only yield about 40 alleles with a 1% VAF, resulting in the limitation of needing to assess NGS panel sensitivity with a small number of alleles.

In addition, in the sensitivity evaluation using the Frampton method, the sensitivity was closely related to two factors: 1) the down-sampled coverage of the *in silico* model corresponding to the read count, and 2) the VAF in the allele. In detail, SNVs with a 5% VAF could be detected 96.1% of the time at 500 coverage, 83.5% at 300 coverage, and 44.2% at 150 coverage. SNVs with a 10% VAF were detected 99.3% of the time at 500 coverage, 98.9% at 300 coverage, and 82.3% at 150 coverage, while SNVs with a VAF greater than 10% could still be detected 99.5% of the time even at 150 coverage. Additionally, specificity was evaluated using the Frampton method. According to the results published in the paper, only when the down-sampled coverage exceeded 500 were two SNVs with a fraction of less than 5% detected; in all other cases, no false positive variants were identified, resulting in a positive predictive value of over 99.9%. This indicates that the specificity of the NGS panel used at that time was very high.

In addition, to detect indels appearing in cancer cell lines using the Frampton method, 41 pools were created by mixing DNA from 2 to 10 cancer cell lines, and the sensitivity and specificity of indel detection were measured. It was confirmed that the detection sensitivity of indels varied depending on: 1) down-sampled coverage and 2) VAF of the indel variants in the mixture. Specifically, indels with a 10% VAF were detected 65% of the time at 190 coverage and 88.3% at 667 coverage. Indels with a 20% VAF were detected 86.3% of the time at 190 coverage and 97.3% at 667 coverage. For false positive indels, no false positive indels with a VAF of 20% or more were detected; however, for indels with a VAF of 20% or lower, approximately 1 to 2% of false positive indels were identified. Similarly, the sensitivity and specificity of gene amplification/deletion detection were assessed using DNA mixed with various cancer cell lines. Although no false positive variants were found, false negative variants were detected at rates of about 10 to 20%.

As described above, the Frampton method faced challenges in accurately assessing the sensitivity of the NGS panel because errors frequently occurred when mixing approximately 10 DNAs in equal amounts during the dilution process. Additionally, it did not provide precise information on the detectability of diluted variants at specific dilution ratios. Furthermore, the detection of indels and amplifications/deletions is generally less sensitive than that of single nucleotide variant (SNV) detection. Establishing specific sensitivity and specificity for NGS panels in guidelines has been difficult due to variables such as allelic bias, duplicate reads, and the detection capabilities of the analysis pipeline (Shin et al., Nature Comm 2017). Therefore, the current guidelines from the American College of Medical Genetics and Genomics for next-generation sequencing-based assays do not establish specific coverage thresholds or minimum sensitivity or specificity [U.S. Food and Drug Administration (FDA). Considerations for Design, Development, and Analytical Validation of Next Generation Sequencing-Based In Vitro Diagnostics Intended to Diagnose Suspected Germline Diseases. Updated 13 April 2018].

However, it provides VAF information that can be detected with a desirable minimum read count at the target site, as well as limit of detection (LoD) information through dilution experiments of standard samples (Rehm et al., Genet Med, 2013; ACMG clinical laboratory standards for next-generation sequencing). Although regulations for NGS methods in this context are not yet well-defined, the American Clinical Laboratory Association (ACLA) currently oversees NGS panels used for genetic testing under the Clinical Laboratory Improvement Amendments (CLIA) rules. The FDA is also in the process of establishing guidelines for the design, development, and analytical validation of these panels, facilitating the clinical application of NGS. Therefore, if new standards and methods are developed to measure the sensitivity and specificity of NGS panels, researchers will be able to compare and evaluate NGS panels more easily, and the minimum standards required for clinical application will also be revised.

### DISCLOSURE

### TECHNICAL PROBLEM

The present invention is directed to providing a composition for validating a next generation sequencing (NGS) panel, comprising homozygote DNA and control genomic DNA.

In addition, the present invention is also directed to providing a kit for validating NGS panels, comprising the composition for validating an NGS panel.

The present invention also provides a method for validating a NGS panel through the analysis of false negative variants, the method comprising: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair allele) from the NGS results; and (c) analyzing the frequency of false negative variants in the DNA mixture samples based on the selected Ho-N pair or He-N pair alleles, wherein the DNA mixture samples are composed of homozygote DNA and control genomic DNA at various dilution ratios.

The present invention also provides a method for validating a NGS panel through the analysis of limit of detection, the method comprising: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair alleles) from the NGS results; (c) analyzing the detection rate of diluted variants in DNA mixture samples based on the selected Ho-N pair alleles or the He-N pair alleles, wherein the DNA mixture samples are composed of homozygote DNA and control genomic DNA at various dilution ratios, and wherein the detection rate of diluted variants is expressed as a percentage of the number of diluted variants found among the Ho-N pair alleles or He-N pair alleles; and (d) analyzing limit of detection.

The present invention also provides a method for validating a NGS panel through the analysis of false positive variants, the method comprising: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) from the NGS result, wherein the N-N pair alleles do not include any variants in either the homozygote DNA or the control genomic DNA; and (c) analyzing the frequency of false positive variants in DNA mixture samples based on the selected N-N pair alleles, wherein the DNA mixture samples are composed of homozygote DNA and control genomic DNA at various dilution ratios.

The present invention also provides a method for providing information for increasing the specificity of a NGS panel, the method comprising: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) homozygote DNA from the result of performing the NGS, wherein the N-N pair alleles do not include any variants in either the homozygote DNA or the control genomic DNA; (c) analyzing the frequency of false positive variants in DNA mixture samples based on the selected N-N pair alleles, wherein the DNA mixture samples are composed of homozygote DNA and control genomic DNA at various dilution ratios; and (d) deriving a stringency cutoff value by analyzing the variant allelic fraction (VAF), which eliminates the false positive variants.

The present invention also provides a method for evaluating errors in the process of generating raw data or errors in the bioinformatics analysis during the NGS panel analysis process of a company, the method comprising: (a) collecting false negative and false positive variants using the bioinformatics analysis results of the company based on the company's raw data; and (b) comparing and analyzing false negative and false positive variants with commercial bioinformatics software using the company's raw data.

### TECHNICAL SOLUTION

In order to achieve the above objects, the present invention provides a composition for validating a next generation sequencing (NGS) panel, comprising homozygote DNA and control genomic DNA.

In an exemplary embodiment of the present invention, the homozygote DNA includes DNA isolated from a hydatidiform mole cell line or a parthenogenetic cell line.

In an exemplary embodiment of the present invention, the control genomic DNA includes genomic DNA isolated from the blood of a normal individual. In addition, the control genomic DNA includes genomic DNA isolated from the blood or cancer tissue of a patient. In addition, the control genomic DNA includes circulating tumor DNA (ctDNA). The control genomic DNA includes synthetic DNA or cloned DNA.

In an exemplary embodiment of the present invention, the dilution ratios of the homozygote DNA and the control genomic DNA are selected from 1:99 to 99:1. Preferably, the dilution ratios of the homozygote DNA to the control genomic DNA are selected from the group consisting of 1:9999, 2.5:9997.5, 5:9995, 1:999, 2.5:997.5, 5:995, 1:99, 2.5:97.5, 5:95, 10:90, 20:80, 50:50, 80:20, 90:10, 95:5, 97.5:2.5, 99:1, 995:5, 997.5:2.5, 999:1, 9995:5, 9997.5:2.5 and 9999:1, but are not limited thereto.

In addition, the present invention provides a kit for validating NGS panels, comprising the composition for validating an NGS panel.

In addition, the present invention provides a method for validating NGS panels through the analysis of false negative variants. The method comprises: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair allele) from the NGS results; and (c) analyzing the frequency of false negative variants in the DNA mixture samples based on the selected Ho-N pair or He-N pair alleles, wherein the DNA mixture samples are composed of homozygote DNA and control genomic DNA at various dilution ratios.

In addition, the present invention provides a method for validating NGS panels through the analysis of limit of detection. The method comprises: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair alleles) from the NGS results; (c) analyzing the detection rate of diluted variants in DNA mixture samples based on the selected Ho-N pair alleles or the He-N pair alleles, wherein the DNA mixture samples are composed of homozygote DNA and control genomic DNA at various dilution ratios, and wherein the detection rate of diluted variants is expressed as a percentage of the number of diluted variants found among the Ho-N pair alleles or He-N pair alleles; and (d) analyzing limit of detection.

In an exemplary embodiment of the present invention, the NGS panel is configured to detect variants selected from the group consisting of single nucleotide variants (SNVs), insertions/deletions (Indels), and chromosomal amplifications/deletions.

In an exemplary embodiment of the present invention, the limit of detection in the analysis is expressed as the lowest variant allelic fraction (VAF) value at which 90% or 95% of the diluted variants are detected in the DNA mixture samples, wherein the DNA mixture samples consist of homozygote DNA and control genomic DNA at various dilution ratios.

In addition, the present invention provides a method for validating NGS panels through the analysis of false positive variants. The method comprises: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) from the NGS result, wherein the N-N pair alleles do not include any variants in either the homozygote DNA or the control genomic DNA; and (c) analyzing the frequency of false positive variants in DNA mixture samples based on the selected N-N pair alleles, wherein the DNA mixture samples are composed of homozygote DNA and control genomic DNA at various dilution ratios.

In an exemplary embodiment of the present invention, the NGS panel is configured to detect variants selected from the group consisting of single nucleotide variants (SNVs), insertions/deletions (Indels), and chromosomal amplifications/deletions.

In addition, the present invention provides a method for enhancing the specificity of NGS panels. The method comprises: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) homozygote DNA from the result of performing the NGS, wherein the N-N pair alleles do not include any variants in either the homozygote DNA or the control genomic DNA; (c) analyzing the frequency of false positive variants in DNA mixture samples based on the selected N-N pair alleles, wherein the DNA mixture samples are composed of homozygote DNA and control genomic DNA at various dilution ratios; and (d) deriving a stringency cutoff value by analyzing the variant allelic fraction (VAF), which eliminates the false positive variants.

In an exemplary embodiment of the present invention, the stringency cutoff value is a 90% at which 90% of false positive variants are removed; a 95% at which 95% of false positive variants are removed; or a 99% at which 99% of false positive variants are removed.

In an exemplary embodiment of the present invention, the method provides information on a stringency cutoff value to enhance the specificity of a NGS panel, wherein the stringency cutoff removes false positive variants.

In addition, the present invention provides a method for evaluating errors in the process of generating raw data or errors in the bioinformatics analysis during the NGS panel analysis process of a company, the method comprising: (a) collecting false negative and false positive variants using the bioinformatics analysis results of the company based on the company's raw data; and (b) comparing and analyzing false negative and false positive variants with commercial bioinformatics software using the company's raw data.

### ADVANTAGEOUS EFFECT

The composition according to the present invention can be effectively usable in the validation of NGS panels. In particular, the validation method of the present invention: (i) analyzes the frequency of false negative variants, the frequency of false positive variants, and the limit of detection for NGS panels, (ii) excludes variants from analysis by identifying high false negative error sites in advance during the validation of the NGS panels, thereby reducing false negative errors in the interpretation of NGS results for specific NGS panels, and (iii) provides an objective stringency cutoff value by analyzing the number of false positive variants and the distribution of variant allelic fraction (VAF), thereby reducing false positive errors in the interpretation of NGS results of the NGS panel.

### BRIEF DESCRIPTION OF THE DRAWINGS

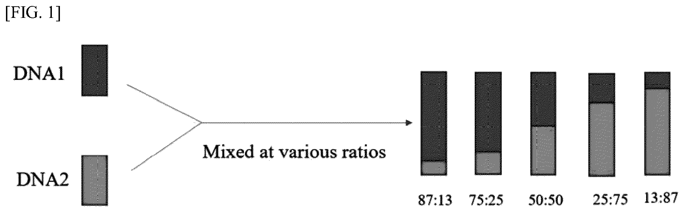

FIG. 1 illustrates that the relative amount of DNA can be measured in DNA mixtures mixed by mixing DNA1 (a homozygous variant) and DNA2 (a null variant) at various ratios.

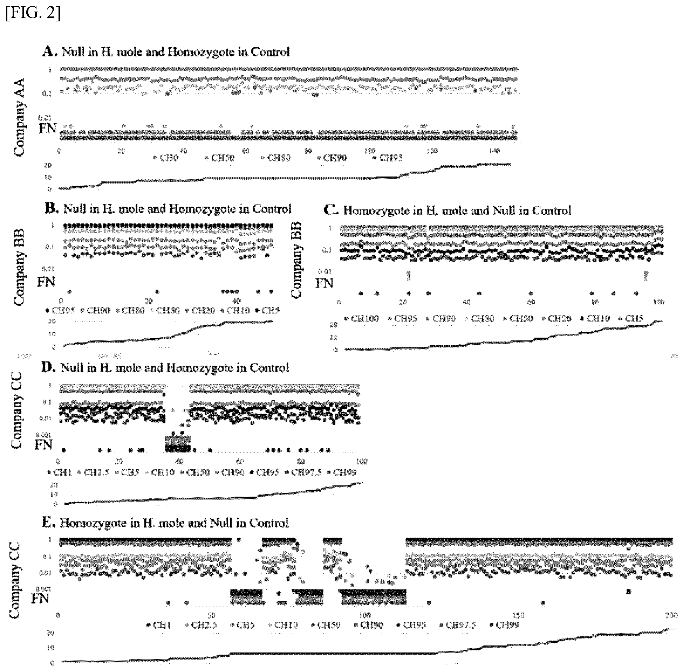

FIG. 2A shows the result of analyzing the limit of detection for the NGS panel of AA Company using Ho-N pair alleles, while FIGS. 2B and 2C show the result for the NGS panel of BB Company. FIGS. 2D and 2E present the result for the NGS panel of CC Company. The X-axis lists alleles by chromosome number and position, and the Y-axis represents Log(VAF), where VAF (variant allelic fraction) is calculated as (variant allele read count)/(total read count). FN indicates false negative alleles, and each color represents the false negative results at the corresponding dilution factor. The X-axis at the bottom of each graph shows the order of alleles by chromosome position, while the Y-axis at the bottom of each graph displays the chromosome number. The dilution ratios of the control genomic DNA and H mole DNA are as follows: CH100, 0:100; CH99, 1:99; CH97.5, 2.5:97.5; CH95, 5:95; CH90, 10:90; CH80, 20:80; CH50, 50:50; CH20, 80:20; CH10, 90:10; CH5, 95:5; CH2.5, 97.5:2.5; CH1, 99:1; and CH0, 100:0.

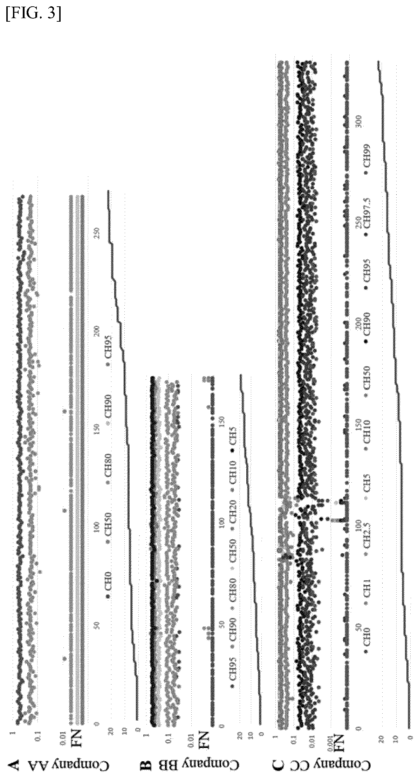

FIG. 3A shows the result of analyzing the limit of detection for the NGS panel of AA Company using He-N pair alleles, while FIG. 3B displays the result for the NGS panel of BB Company, and FIG. 3C shows the result for the NGS panel of CC Company. The X-axis lists alleles in order by chromosome number and position, and the Y-axis represents Log(VAF), where VAF (variant allelic fraction) is calculated as (variant allele read count)/(total read count). FN indicates false negative alleles, with each color representing the false negative results at the corresponding dilution factor. The X-axis at the bottom of each graph shows the list in order by chromosome position, while the Y-axis at the bottom of each graph displays the chromosome number. The dilution ratios of the control genomic DNA and H mole DNA are as follows: CH99, 1:99; CH97.5, 2.5:97.5; CH95, 5:95; CH90, 10:90; CH80, 20:80; CH50, 50:50; CH20, 80:20; CH10, 90:10; CH5, 95:5; CH2.5, 97.5:2.5; CH1, 99:1; and CH0, 100:0.

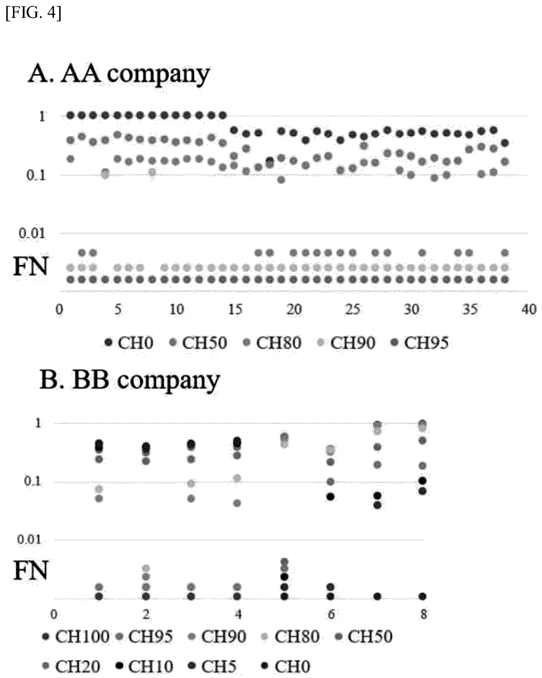

FIG. 4A shows the result of analyzing indel detection according to the dilution ratios for the NGS panel of AA Company using Ho-N pair alleles or He-N pair allele. FIG. 4B displays the result of indel detection analysis based on the dilution ratios for the NGS panel of BB Company. The X-axis lists alleles in order, while the Y-axis represents Log(VAF), where VAF (variant allelic fraction) is calculated as (variant allele read count)/(total read count). FN indicates false negative alleles, with each color representing the false negative results at the corresponding dilution factor. The dilution ratios of the control genomic DNA and H mole DNA are as follows: CH100, 0:100; CH95, 5:95; CH90, 10:90; CH80, 20:80; CH50, 50:50; CH20, 80:20; CH10, 90:10; CH5, 95:5; and CH0, 100:0.

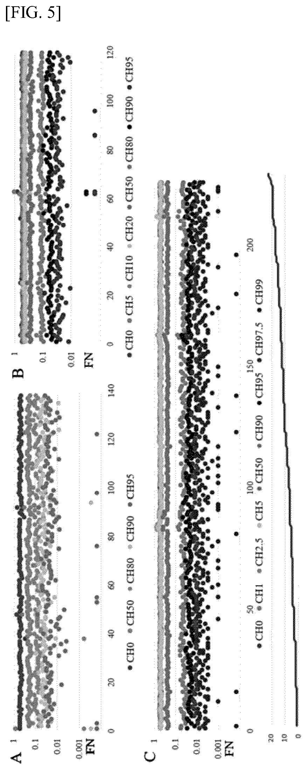

FIG. 5A shows the result of analyzing the limit of detection for the NGS panel of AA Company using Ho-He pair alleles, while FIG. 5B shows the result for the NGS panel of BB Company, and FIG. 5C displays the result for the NGS panel of CC Company. The X-axis lists alleles by chromosome position, and the Y-axis represents Log(VAF), where VAF (variant allelic fraction) is calculated as (variant allele read count)/(total read count). FN indicates false negative alleles, and each color represents the false negative results at the corresponding dilution factor. The X-axis at the bottom of the graph in FIG. 5C lists the alleles in order by chromosome position, while the Y-axis at the bottom of the same graph displays the chromosome number. The dilution ratios of the control genomic DNA and H mole DNA are as follows: CH100, 0:100; CH99, 1:99; CH97.5, 2.5:97.5; CH95, 5:95; CH90, 10:90; CH80, 20:80; CH50, 50:50; CH20, 80:20; CH10, 90:10; CH5, 95:5; CH2.5, 97.5:2.5; CH1, 99:1; and CH0, 100:0.

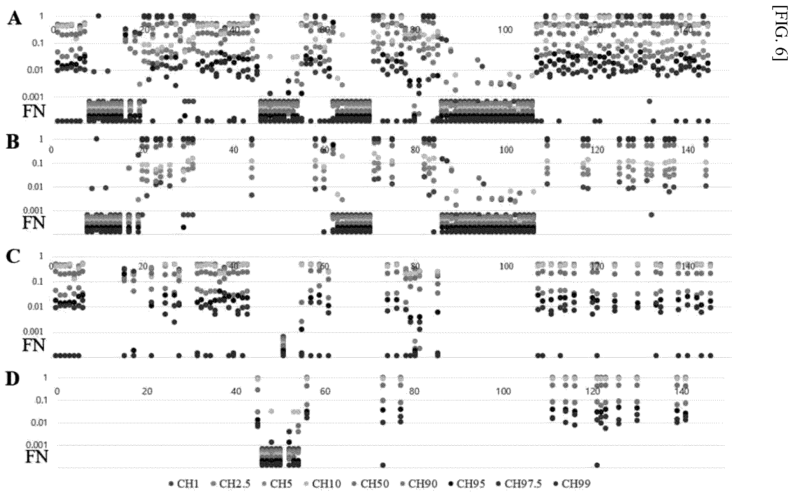

FIG. 6A shows the result of analyzing the limit of detection for chromosome 6 variants in Ho-N pair alleles or He-N pair alleles for the NGS panel of CC Company, FIG. 6B shows the result of analyzing the limit of detection of chromosome 6 variants in Ho-N pair alleles that are null in control DNA, FIG. 6C shows the result of analyzing the limit of detection for chromosome 6 variants in He-N pair alleles, and FIG. 6D shows the result of analyzing the limit of detection for chromosome 6 variants in Ho-N pair alleles that are homozygous in control DNA. The X-axis lists alleles by chromosome position, and the Y-axis represents Log(VAF), where VAF (variant allelic fraction) is calculated as (variant allele read count) / (total read count). FN indicates false negative alleles, and each color represents the false negative results at the corresponding dilution factor. The dilution ratios of the control genomic DNA and H mole DNA are as follows: CH99, 1:99; CH97.5, 2.5:97.5; CH95, 5:95; CH90, 10:90; CH50, 50:50; CH10, 90:10; CH5, 95:5; CH2.5, 97.5:2.5; and CH1, 99:1.

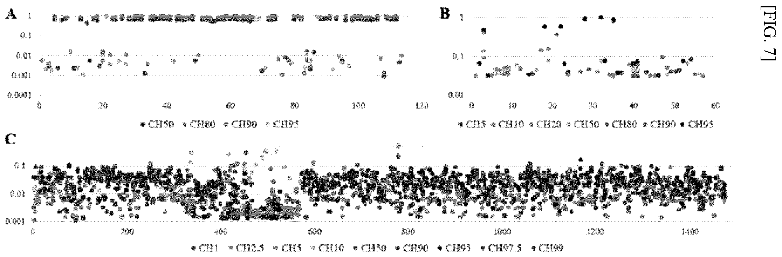

FIG. 7A shows the result of analyzing the frequency of false positive variants in the NGS panel of AA Company using N-N pair alleles, where both the H mole DNA and the control genomic DNA are null. FIG. 7B shows the result for the NGS panel of BB Company, while FIG. 7C displays the result for the NGS panel of CC Company. The X-axis lists alleles in order by chromosome position, and the Y-axis represents Log(VAF), where VAF (variant allelic fraction) is calculated as (variant allele read count) / (total read count). The dilution ratios of the control genomic DNA and H mole DNA are as follows: CH99, 1:99; CH97.5, 2.5:97.5; CH95, 5:95; CH90, 10:90; CH50, 50:50; CH10, 90:10; CH5, 95:5; CH2.5, 97.5:2.5; and CH1, 99:1.

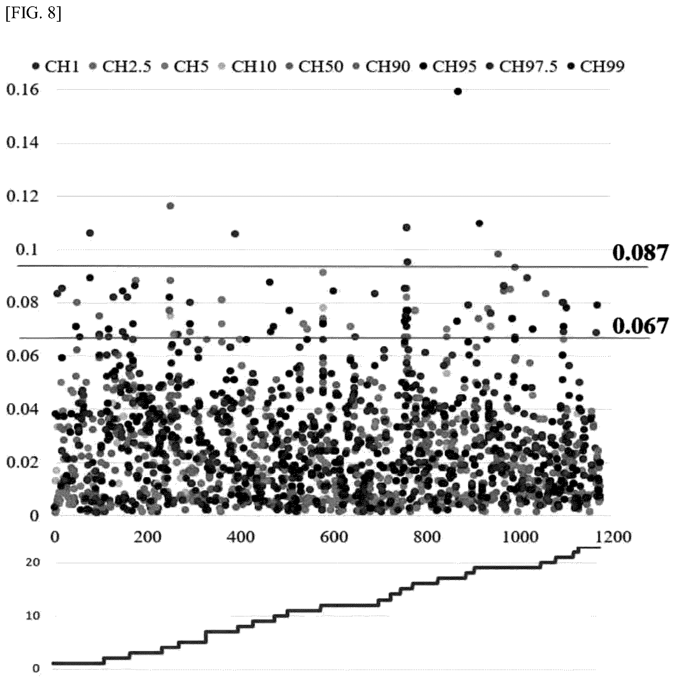

FIG. 8 shows the result of deriving the stringency cutoff value by removing the false positive variants of chromosome 6 from the NGS panel result of CC Company and shows only the remaining false positive variants. The X-axis lists alleles in order by chromosome position, while the Y-axis represents Log(VAF), where VAF (variant allelic fraction) is calculated as (variant allele read count) / (total read count). The dilution ratios of the control genomic DNA and H mole DNA are as follows: CH99, 1:99; CH97.5, 2.5:97.5; CH95, 5:95; CH90, 10:90; CH50, 50:50; CH10, 90:10; CH5, 95:5; CH2.5, 97.5:2.5; and CH1, 99:1.

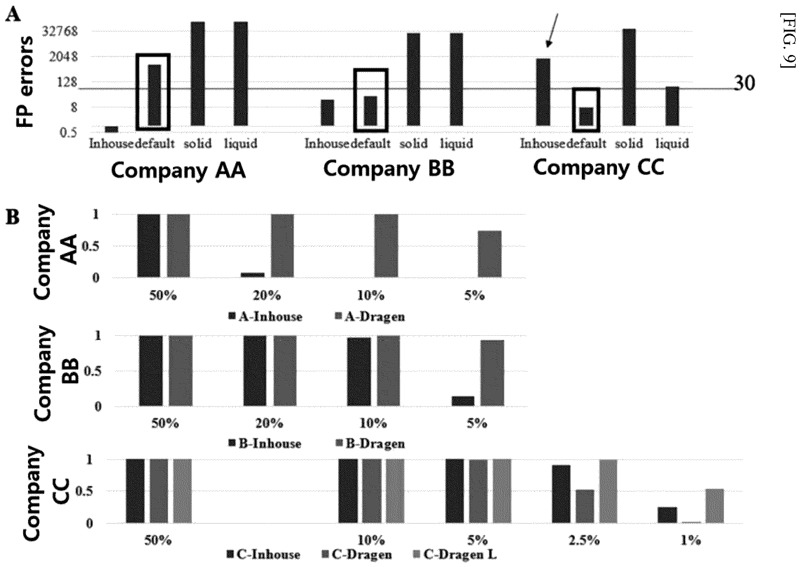

FIG. 9A shows the results of analyzing the false positive variants among the derived variants. This includes the analysis of false positive variants from each company's results (In-house) and the analysis from variants derived using DRAGEN software under three conditions: default, solid, and liquid. The Y-axis represents the arithmetic mean value of false positive variants found in multiple diluted samples. FIG. 9B shows the results of measuring sensitivity by analyzing false negatives from the variant detection results of the NGS panels of companies AA, BB, and CC. and the results of analyzing the false negatives of variants derived from each company's respective bioinformatics methods (In-house) and the results of analyzing the false negatives of variants derived from Dragen software's bioinformatics method under three conditions (default, solid, and liquid) are displayed in a graph (y-axis) for the dilution variant detection rate (x-axis: % is the % of samples with variants, so the % of total DNA in each sample that is variants is half of the % of variant samples). To determine false positive errors, all cases where a variant or reference nucleotide was found in the alleles that were not present in either the H mole DNA or the control genomic DNA were classified as false positive errors.

### MODES OF THE INVENTION

Hereinafter, the present invention will be described in more detail.

The terms used in the present invention were selected from the most commonly employed general terms, taking into account the functions of the invention. However, these terms may vary depending on the preferences of engineers working in the relevant technical field or the emergence of new technologies. Moreover, in certain instances, terms are chosen arbitrarily, and their meanings will be explained in detail in the description section of the relevant embodiment. Therefore, the terms used in the present invention should be defined based on their contextual meanings and the overall content of the invention, rather than merely by their names.

When the phrase "include or comprise" is used to refer to a component or step in the present invention, it does not exclude other components or steps unless specifically stated otherwise. Instead, it indicates that additional components or steps may also be included.

The present invention provides a composition for validating a next generation sequencing (NGS) panel, comprising homozygote DNA and control genomic DNA.

The term "next-generation sequencing" in the present invention refers to a technology that can analyze millions of nucleotide sequences simultaneously, also known as massively parallel sequencing or high-throughput sequencing. In this invention, next-generation sequencing and NGS are used interchangeably.

The term "allele" in the present invention refers to the DNA sequence of an allelic gene, which is a gene that forms a pair of homologous chromosomes and exhibits different characteristics. Homologous chromosomes can be classified as homozygous and heterozygous; "homozygotes" are combinations of alleles with the same properties, while "heterozygotes" are combinations of alleles with different properties. In the present invention, the terms "allelic gene" and "allele" are used interchangeably.

In the present invention, the composition contains two different genomic DNAs to validate the NGS panel, and one of the DNAs uses homozygote DNA.

In the present invention, the homozygote DNA includes DNA isolated from a hydatidiform mole cell line or a parthenogenetic cell line, but is not limited thereto.

The term "control genomic DNA" of the present invention means one genomic DNA other than homozygote DNA among two different genomic DNAs included in the composition. This control genomic DNA may be a homozygote or heterozygous genomic DNA.

In the present invention, the control genomic DNA includes genomic DNA isolated from the blood of a normal individual. In addition, the control genomic DNA includes genomic DNA isolated from the blood or cancer tissue of a patient. In addition, the control genomic DNA includes circulating tumor DNA (ctDNA). The control genomic DNA includes synthetic DNA or cloned DNA.

In the present invention, for the composition, the dilution ratios of the homozygote DNA and the control genomic DNA are selected from 1:99 to 99:1, and preferably, the dilution ratios of the homozygote DNA to the control genomic DNA are selected from the group consisting of 1:9999, 2.5:9997.5, 5:9995, 1:999, 2.5:997.5, 5:995, 1:99, 2.5:97.5, 5:95, 10:90, 20:80, 50:50, 80:20, 90:10, 95:5, 97.5:2.5, 99:1, 995:5, 997.5:2.5, 999:1, 9995:5, 9997.5:2.5 and 9999:1, but is not limited thereto.

In addition, the present invention provides a kit for validating an NGS panel, comprising the composition for validating an NGS panel.

In addition, the present invention provides a method for validating NGS panels through the analysis of false negative variants. The method comprises: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair allele) from the NGS results; and (c) analyzing the frequency of false negative variants in the DNA mixture samples based on the selected Ho-N pair or He-N pair alleles, wherein the DNA mixture samples are composed of homozygote DNA and control genomic DNA at various dilution ratios.

The term "false negative" in the present invention refers to a case where a test result that should have been originally positive is erroneously negative.

In addition, the present invention provides a method for validating NGS panels through the analysis of limit of detection. The method comprises: (a) performing NGS using a NGS panel on DNA mixture samples, wherein the DNA mixture samples consist of homozygote DNA and control genomic DNA at various dilution ratios; (b) selecting Ho-N pair alleles (Homozygote-Null pair alleles) or He-N pair alleles (Heterozygote-Null pair alleles) from the NGS results; (c) analyzing the detection rate of diluted variants in DNA mixture samples based on the selected Ho-N pair alleles or the He-N pair alleles, wherein the DNA mixture samples are composed of homozygote DNA and control genomic DNA at various dilution ratios, and wherein the detection rate of diluted variants is expressed as a percentage of the number of diluted variants found among the Ho-N pair alleles or He-N pair alleles; and (d) analyzing limit of detection.

The term "Ho-N pair allele (Homozygote-Null pair allele)" of the present invention refers to an allele in which one allelic site is mixed with a homozygous variant and a null without variant.

The term "He-N pair allele (Heterozygote-Null pair allele)" of the present invention refers to an allele in which one allelic site is mixed with a heterozygous variant and a null without variant.

The term "dilution variant detection rate" of the present invention refers to the number of diluted variants, expressed as a percentage, found among Ho-N pair alleles or He-N pair alleles in the DNA mixture samples, where homozygote DNA and control genomic DNA are mixed at different dilution ratios.

In the present invention, the NGS panel is configured to detect variants selected from the group consisting of single nucleotide variants (SNVs), insertions/deletions (Indels), and chromosomal amplifications/deletions.

The term "variant" in the present invention means a modification of a chromosome, gene or nucleotide sequence that is genetically distinct from the wild type. In the present invention, *byeonyi* (which is transliteration of Korean pronunciation) or variant is interchangeably described.

In the present invention, the limit of detection in the analysis is expressed as the lowest variant allelic fraction (VAF) value at which 90% or 95% of the diluted variants are detected in the DNA mixture samples, wherein the DNA mixture samples consist of homozygote DNA and control genomic DNA at various dilution ratios.

In the present invention, for validating the NGS panel, a composition for validation of the NGS panel was used, which includes a DNA mixture samples containing DNA isolated from a hydatidiform mole cell line, which is homozygote DNA (hereinafter, "H mole DNA"), and control genomic DNA at various dilution ratios.

For example, if there is DNA1 containing a homozygous variant at one allelic site and DNA2 containing a null at that site, mixing DNA1 and DNA2 allows for the calculation of the variant allelic fraction (VAF). This enables the determination of the dilution ratios of DNA1 and DNA2. The limit of detection can then be derived by expressing the VAF corresponding to the maximum dilution that results in a detection rate of diluted variants of 90% or 95% as a percentage.

More specifically, FIG. 1 illustrates that the relative amount of mixed DNA can be quantified in each DNA mixtures where homozygous variant DNA1 and null DNA2 are mixed at various ratios. In this context, an allele where one allelic site is associated with a homozygous variant and the other is a null is termed a "Ho-N pair allele (Homozygous-Null pair allele)." The probability of the appearance of Ho-N pair alleles in various alleles (alleles 1~9 in Table 1) each of which has various variant allelic fraction (VAF) is presented in Table 1 below.

**[Table 1]**

| | Allele 1 | Allele 2 | Allele 3 | Allele 4 | Allele 5 | Allele 6 | Allele 7 | Allele 8 | Allele 9 | Sum |
|---|---|---|---|---|---|---|---|---|---|---|
| p | 0.1 | 0.2 | 0.3 | 0.4 | 0.5 | 0.6 | 0.7 | 0.8 | 0.9 | |
| q (= 1-p) | 0.9 | 0.8 | 0.7 | 0.6 | 0.5 | 0.4 | 0.3 | 0.2 | 0.1 | |
| PL = 2*p²q² | 0.0162 | 0.0512 | 0.0882 | 0.1152 | 0.125 | 0.1152 | 0.0882 | 0.0512 | 0.0162 | 0.6666 |
| P2 = pq | 0.09 | 0.16 | 0.21 | 0.24 | 0.25 | 0.24 | 0.21 | 0.16 | 0.09 | 1.65 |

| | | | | | | | | | | |
|---|---|---|---|---|---|---|---|---|---|---|
| * p = VAF (variant allelic fraction); q = 1-p; P1: P1: probability that the Ho-N pair allele will appear when both DNAs are common genomic DNA (P1 = p²q²+q²p² = 2 p²q²); and P2: probability that the Ho-N pair allele will appear when one of the two DNAs is the H mole DNA (P2 = p²q + q²p = pq(p + q) = pq). | | | | | | | | | | |

As shown in Table 1 above, when calculating and comparing the number of Ho-N pair alleles from alleles with nine different VAF (variant allelic fraction, p), we observe the following: in the scenario where both DNAs are heterozygous genomic DNA, the number of Ho-N pair alleles among nine alleles with various VAFs is calculated to be 0.67. In contrast, in the scenario where one of the DNA mixtures is H mole DNA (homozygous DNA) and the other one is heterozygous genomic DNA, it is calculated to be 1.65. This indicates that when one DNA is H mole DNA, the number of Ho-N pair alleles can be obtained at a rate approximately 2.45 times higher (i.e., the P2/P1 ratio) than when both DNAs are heterozygous genomic DNA.

In particular, when calculating the probability by distinguishing between cases where the variant in each allele is analyzed as an actual variant and cases where it is analyzed as a reference, the P2/P1 ratio remained unchanged. Furthermore, even when the frequency of the allele classified as the reference allele was lower than that of the variant allele, the P2/P1 ratio remained the same. Additionally, when calculating the frequency of the variant appearing in the distribution of each allele and integrating the p value in the range of 0 to 1, the P2/P1 ratio showed little variation, remaining around 2.50 (for example, considering that the frequency of allele 1 is 0.1 and the frequency of allele 9 is 0.9).

Accordingly, in the present invention, when one of the DNAs in the two-DNA mixtures is H mole DNA (homozygote DNA), the number of Ho-N pair alleles is about 2.5 times greater than in other scenarios. Thus, it was confirmed that using H mole DNA, which is homozygous DNA, may be more advantageous for analyzing the limit of detection of the NGS panel.

In addition, the present invention provides a method for validating NGS panels through the analysis of false positive variants. The method comprises: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) from the NGS result, wherein the N-N pair alleles do not include any variants in either the homozygote DNA or the control genomic DNA; and (c) analyzing the frequency of false positive variants in DNA mixture samples based on the selected N-N pair alleles, wherein the DNA mixture samples are composed of homozygote DNA and control genomic DNA at various dilution ratios

The term "N-N pair allele (Null-Null pair allele)" of the present invention refers to an allele in which all allelic sites are composed of null without variant.

The term "false positive" in the present invention refers to a case where a test result that should have been originally negative is erroneously positive.

In the present invention, if a variant is found to exist in each DNA mixture samples in which homozygote DNA and control genomic DNA are mixed at different dilution ratios based on an N-N pair allele in which both the homozygote DNA and the control genomic DNA are null, it is judged to be a false positive variant.

In the present invention, the NGS panel is configured to detect variants selected from the group consisting of single nucleotide variants (SNVs), insertions/deletions (Indels), and chromosomal amplifications/deletions.

In addition, the present invention provides a method for enhancing the specificity of NGS panels. The method comprises: (a) performing NGS using a NGS panel on DNA mixture samples, wherein the DNA mixture samples consist of homozygote DNA and control genomic DNA at various dilution ratios; (b) selecting N-N pair alleles (Null-Null pair alleles) homozygote DNA from the result of performing the NGS, wherein the N-N pair alleles do not include any variants in either the homozygote DNA or the control genomic DNA; (c) analyzing the frequency of false positive variants in DNA mixture samples based on the selected N-N pair alleles, wherein the DNA mixture samples are composed of homozygote DNA and control genomic DNA at various dilution ratios; and (d) deriving a stringency cutoff value by analyzing the variant allelic fraction (VAF), which eliminates the false positive variants.

In the present invention, the stringency cutoff value is a 90% at which 90% of false positive variants are removed; a 95% at which 95% of false positive variants are removed; or a 99% at which 99% of false positive variants are removed.

In the present invention, the method provides information on a stringency cutoff value to enhance the specificity of a NGS panel, wherein the stringency cutoff removes false positive variants.

Hereinafter, the present invention will be described in more detail through specific examples. These examples are intended to illustrate the present invention, and the scope of the present invention is not limited to these examples.

### Example 1. Preparation of DNA mixture and NGS analysis

In the present invention, the sensitivity and specificity of the NGS panels from three companies were compared and analyzed using H mole DNA, which is homozygote DNA, to validate the NGS panels. The H mole DNA, derived from human hydatidiform mole, was purchased from Coriell Company (NA07489, Camden, NJ), while the control genomic DNA was extracted from the blood of a co-researcher with the approval of the Institutional Review Board of the National Cancer Center, Korea.

The prepared H mole DNA, control genomic DNA, and the DNA mixture containing H mole DNA and control genomic DNA were sent to companies AA, BB, and CC, respectively. Experiments were performed using the Novaseq 6000 NGS equipment from Illumina Company and the NGS panel of each company. Following the experiments, the validation of NGS panel was performed by receiving files that analyzed the variants using the method provided by each company from the FASTQ files obtained during the experiment. However, the number of variants obtained from each company's NS panel differs depending on each company's analysis method, for example, the number of identified genetic variants. However, all companies commonly used the hg19 of the NCBI human genome assembly as a reference, and CC Company employed both LoFreq and MuTect as variant callers to analyze more variants.

### Example 2. NGS Validation Method

### 2.1. Selection of Ho-N pair allele and He-N pair allele

The results of the variants analyzed by each company were sorted based on the chromosome number and position, along with each sample information, such as reference base, variant allele information, VAF, read count, and information on the variants. In the analysis results of companies AA and BB, any variant with a read count of less than 100 was excluded, while in the analysis results of company CC, variants with the read count less than 300 were excluded. In addition, for companies AA and BB, the analysis included SNVs in both exon and intron sites of target genes. For CC Company, however, the analysis was limited to the exon site SNVs and splice site SNVs while excluding intron site SNVs.

Among the selected alleles, a pair in which the allele from the H mole DNA and the control genomic DNA consists of a homozygous variant and a null allele was defined as a "Ho-N pair allele (Homozygote-Null pair allele)", and a pair in which the H mole DNA is null and the control genomic DNA is a heterozygous variant was defined as a "He-N pair allele (Heterozygote-Null pair allele)."

### 2.2. Determination of false negative variant, dilution variant detection rate, and limit of detection using Ho-N pair allele and He-N pair allele

An allele in which a variant is present in either the H mole DNA (homozygote DNA) or the control genomic DNA, but is not detected in the NGS panel analysis of the DNA mixture samples, is defined as a false negative allele. The frequency of false negative alleles was analyzed by categorizing the alleles into Ho-N pair alleles and He-N pair alleles within the DNA mixture samples.

The detection rate of diluted variants was defined as the percentage of the detected variants among the Ho-N pair alleles or He-N pair alleles in DNA mixtures.

The limit of detection (LoD) was defined as the percentage of the variant allelic fraction (VAF) corresponding to the maximum dilution ratios that results in the detection rate of diluted variants of 90% or 95% among DNA mixtures. In this context, VAF is calculated by dividing the read count of the allele detected as a variant at the specific allele position by the total read count, which is obtained by adding the counts of alleles detected as both variants and reference bases at that specific allele position.

### 2.3. Determination of false positive variant and stringency cutoff value

Among the null alleles in which no variant was found in either the H mole DNA or the control genomic DNA, if the NGS analysis results of the DNA mixture samples indicate the presence of a variant, it is classified as a false positive variant. The variant allelic fraction (VAF) value that effectively removes 95% or 99% of these false positive variants is defined as the 95% stringency cutoff value or the 99% stringency cutoff value, respectively.

### Example 3. Sensitivity analysis results for NGS panel using Ho-N pair allele

Among all the variant sites from the results of companies AA, BB, and CC, the median read counts of the variant alleles were confirmed to be 197 (Q1, Q3; 51,464), 813 (433, 1189), and 679 (577, 717), respectively. In contrast, the median read counts in the samples for the Ho-N pair allele and He-N pair allele of the present invention were confirmed as 380 (Q1, Q2; 221, 633) in AA Company, 901 (Q1, Q; 503, 1234) in BB Company, and 703 (Q1, Q2; 679, 727) in CC Company, respectively.

First, the sensitivity of the NGS panels of three companies was evaluated by analyzing the Ho-N pair allele. Since the variant analysis results from AA Company showed that there were almost no alleles in the exon sites, the sensitivity and specificity were assessed including intron site variants. As a result of selecting Ho-N pair alleles from the analysis results of AA Company, fifteen (10.2%, 15/147) were identified as exon site variants, and the remainder were identified as intronic site or UTR site variants.

In addition, the results of variant analysis from BB included both SNV and indel, and the sensitivity and specificity were analyzed with consideration of both exon and intron variants. As a result of selecting the Ho-N pair alleles, 134 (89.9%, 134/149) were confirmed as exon site variants or splice site variants. In the variant analysis results from CC Company, only exon site variants and splice site variants were selected because the number of analyzed alleles was relatively large; indels were removed, and only SNVs were analyzed. A total of 306 Ho-N pair alleles were confirmed.

As a result of analyzing the sensitivity of each company's NGS panel using Ho-N pair allele, it was found that the NGS panel of AA Company detected all SNVs in the DNA mixture (CH50) containing 50% H mole DNA and 50% control genomic DNA. Additionally, 134 out of 147 Ho-N pair alleles were detected in the DNA mixture (CH80) containing 80% H mole DNA and 20% control genomic DNA, confirming a detection rate of diluted variants of 91.2% (FIG. 2A). In contrast, only 16 SNVs were detected in the DNA mixture (CH90) containing 90% H mole DNA and 10% control genomic DNA, resulting in a detection rate of diluted variants of 10.9% (FIG. 2A). Consequently, the maximum dilution ratios showing a 90% detection rate for diluted variants in AA Company's NGS panel was confirmed to be approximately 20%, indicating that the limit of detection for achieving a 90% detection rate of diluted variants is approximately 20%.

The BB Company's NGS panel detected all 149 Ho-N pair alleles in the 10% DNA mixture samples (i.e., the DNA mixture (CH90) containing 90% H mole DNA and 10% control genomic DNA and DNA mixture (CH10) containing 10% H mole DNA and 90% control genomic DNA). It also detected 137 out of 149 Ho-N pair alleles in the 5% DNA mixture samples (i.e., the DNA mixture (CH95) containing 95% H mole DNA and 5% control genomic DNA and the DNA mixture (CH5) containing 5% H mole DNA and 95% control genomic DNA), confirming a dilution variant detection rate of approximately 88.6% (FIGS. 2B and 2C). Accordingly, the maximum dilution ratios showing a 90% detection rate for diluted variants in BB Company's NGS panel was confirmed to be between 5 and 10%, and more closely approximately 5%. This indicates that the limit of detection showing a 90% detection rate of diluted variants is approximately 5%.

In the results from CC Company, some genes on chromosome 6 exhibited a high number of false negatives, leading to their exclusion from the limit of detection analysis. Chromosome 6 contains 91 Ho-N pair alleles, so excluding these, a total of 215 Ho-N pair alleles were analyzed. The CC Company's NGS panel detected all 215 Ho-N pair alleles in the 2.5% DNA mixture samples (i.e., the DNA mixture (CH97.5) containing 97.5% H mole DNA and 2.5% control genomic DNA, as well as the DNA mixture (CH2.5) containing 2.5% H mole DNA and 97.5% control genomic DNA). In addition, it detected 197 out of 215 Ho-N pair alleles in the 1% DNA mixture samples (i.e., the DNA mixture (CH99) containing 99% H mole DNA and 1% control genomic DNA, and the DNA mixture (CH1) containing 1% H mole DNA and 99% control genomic DNA), confirming a dilution variant detection rate of approximately 91.6% (FIGS. 2D and 2E). Consequently, the maximum dilution ratios exhibiting a 90% detection rate for diluted variants in CC Company's NGS panel was confirmed to be 1%, indicating that the limit of detection for achieving a 90% detection rate of diluted variants is approximately 1%.

From the above results, it was confirmed that the Ho-N pair alleles can be selected using the DNA mixture containing the H mole DNA of the present invention. Additionally, the limit of detection, expressed as a percentage of the variant allelic fraction (VAF) corresponding to the maximum dilution ratios that shows a 90% dilution variant detection rate, can be analyzed. This allows for the validation of the NGS panel of each company's NGS panel through the analysis of the frequency of false negative variants and the analysis of the limit of detection.

### Example 4. Sensitivity analysis results for NGS panel using He-N pair allele

The sensitivity was analyzed to determine whether it could be assessed using He-N pair alleles. The analysis results from AA Company revealed 269 He-N pair alleles, of which 53 were exonic variants, accounting for 19.7% of the total. The AA Company's NGS panel detected 266 out of 269 He-N pair alleles in the 50% DNA mixture sample (CH50), confirming a detection rate of diluted variants of 98.9% (FIG. 3A). This indicates that 98.9% of variant alleles with a variant allelic fraction (VAF) of 0.25 were detected, suggesting that the limit of detection is less than or equal to 25%. The results were confirmed to be similar to those obtained using the previously analyzed Ho-N pair alleles.

The analysis results from BB Company revealed 172 He-N pair alleles, of which 147 were exonic variants or splice variants, accounting for 85.5% of the total. The BB Company's NGS panel detected 166 out of 172 He-N pair alleles in the 10% DNA mixture samples (i.e., the DNA mixture (CH90) containing 90% H mole DNA and 10% control genomic DNA, and the DNA mixture (CH10) containing 10% H mole DNA and 90% control genomic DNA), confirming a detection rate of diluted variants of 96.5% (FIG. 3B). This indicates that 96.5% of variant alleles with a variant allelic fraction (VAF) of 0.05 were detected, suggesting that the limit of detection is less than or equal to 5%. The results were found to be similar to those obtained using the previously analyzed Ho-N pair alleles

The analysis results from CC Company identified 276 He-N pair alleles after excluding 54 He-N pair alleles present on chromosome 6 (which were all exon site SNVs). The CC Company's NGS panel detected 246 out of the 276 He-N pair alleles in the 2.5% DNA mixture samples (i.e., the DNA mixture (CH97.5) containing 97.5% H mole DNA and 2.5% control genomic DNA, and the DNA mixture (CH2.5) containing 2.5% H mole DNA and 97.5% control genomic DNA), confirming a detection rate of diluted variants of 89.1% (FIG. 3C). This indicates that 89.1% of variant alleles with a variant allelic fraction (VAF) of 0.0125 were detected, suggesting that the limit of detection is 1.25%. The results were found to be similar to those obtained using the previously analyzed Ho-N pair alleles.

For reference, among the Ho-N and He-N pair alleles from companies AA and BB, 38 and 8 indels were included, respectively, in addition to SNVs. These indels had minimal impact on the calculation of the limit of detection (FIG. 4).

From the above results, it was confirmed that the present invention can objectively analyze the limit of detection for each NGS panel by determining the maximum dilution ratios that shows a 90% detection rate of diluted variants through the analysis of Ho-N and He-N pair alleles. Thus, it can be effectively utilized for the validation of NGS panels. Additionally, it was noted that using both the results of Ho-N pair alleles and He-N pair alleles provides the advantage of obtaining sensitivity information for more alleles compared to using only the Ho-N pair alleles.

### Example 5. Relevance between limit of detection and read count or VAF (variant allelic fraction)

In a prior document, it was reported that for SNV detection, when the read count (or local sequence coverage) is 350, alleles with a VAF of less than 5% can be detected at 89.4%, while alleles with a VAF of 5 to 10% can be detected at 99.2%. In contrast, when the read count is 738, alleles with a VAF of less than 5% can be detected at 98.5%, and alleles with a VAF of 5 to 10% can be detected at 99.7%. This indicates that both read count and VAF are correlated with the detection sensitivity or limit of detection of variants (Frampton et al., Nature Biotechnology 2013).

However, in the actual analysis results, although the median read count for AA Company was approximately 380, it detected only 10.9% of variants with a VAF of 10% and 91.2% of variants with a VAF of 20%, indicating that the sensitivity of AA Company's NGS panel was significantly low. In contrast, BB Company achieved a read count of approximately 900, detecting 88.6% of variants with a VAF of 5%, suggesting that the limit of detection for BB Company's NGS panel was consistent with the results from the aforementioned document.

However, CC Company had a read count of about 700 and detected 100% of variants with a VAF of 2.5%, along with 91.6% of variants with a VAF of 1%. This indicates that the limit of detection for CC Company's NGS panel is superior to the results reported in the aforementioned document. Moreover, these findings suggest that multiple variables may influence the sensitivity of NGS panels beyond just read count and VAF. Therefore, it can be concluded that the sensitivity or limit of detection of the NGS panel cannot be evaluated solely based on read count and VAF. Researchers need to validate the sensitivity of each NGS panel using standard references to determine whether a specific NGS panel is sensitive enough to meet the requirements of their research purposes.

### Example 6. Sensitivity analysis results for NGS panel using Ho-He pair allele

Next, a pair consisting of a homozygous variant in H mole DNA and a heterozygous variant in the control genomic DNA was defined as a "Ho-He pair allele (Homozygote and Heterozygous pair allele)", and the RAF (reference allelic fraction) value, which represents the fraction of the diluted reference allele compared to the variant allele, was obtained, and it was assessed whether the limit of detection could be analyzed based on this value.

In the analysis using the RAF, AA Company's NGS panel detected 94.9% (131/138) of Ho-He pair alleles with a 2.5% RAF in a 5% DNA mixture sample (i.e., a DNA mixture (CH95) containing 95% H mole DNA and 5% control genomic DNA) (FIG. 5A). In addition, BB Company's NGS panel detected 96.7% (116/120) of Ho-He pair alleles with a 2.5% RAF in 5% DNA mixture samples (i.e., a DNA mixture (CH95) containing 95% H mole DNA and 5% control genomic DNA, and a DNA mixture (CH5) containing 5% H mole DNA and 95% control genomic DNA) (FIG. 5B). Furthermore, CC Company's NGS panel detected 97.8% (221/226) of Ho-He pair alleles with a 0.5% RAF in 1% DNA mixture samples (i.e., a DNA mixture (CH99) containing 99% H mole DNA and 1% control genomic DNA, and a DNA mixture (CH1) containing 1% H mole DNA and 99% control genomic DNA) (FIG. 5C).

As described above, the results of the analysis of the limit of detection using the Ho-He pair alleles demonstrated that the detection rate of the diluted reference bases was 90% or higher at the maximum dilution ratios by each company. In addition, false negative alleles on chromosome 6 identified in CC Company's NGS panel were not clearly detected.

As described above, the analysis results using the Ho-He pair alleles were found to be significantly different from the results of diluted variant detection analysis obtained using Ho-N pair alleles or He-N pair alleles. Therefore, it was confirmed that the method of analyzing the limit of detection of reference bases using the RAF values of Ho-He pair alleles is not suitable for NGS panel evaluation.

### Example 7. Additional analysis result of limit of detection for chromosome 6

The CC Company's NGS panel was found to have a significant issue with numerous false negatives on chromosome 6, and the analysis using He-N pair alleles yielded relatively lower results compared to the analysis using Ho-N pair alleles. Consequently, the regions on chromosome 6 with many false negatives were matched to the chromosomal positions of the Ho-N pair alleles or He-N pair alleles, and it was subsequently assessed whether there were any differences in the analysis results obtained using the Ho-N pair alleles versus the He-N pair alleles.

As a result, it was confirmed that certain genetic sites within chromosome 6 exhibited a higher frequency of false negatives, and the He-N pair alleles contained fewer of these genetic sites, leading to fewer false negatives compared to the Ho-N pair alleles (FIG. 6). Similar to the findings with CC Company's NGS panel, the presence of specific chromosomal sites associated with false negatives suggests that even mutations with a large variant allelic fraction (VAF) could still result in false negatives.

From the above results, it was confirmed that the method of the present invention has the advantage of allowing for the determination of the final result while accounting for the possibility of false negatives at relevant sites during analysis. This is achieved by identifying the presence or absence of specific chromosomal sites or gene sites where false negatives occur frequently during the validation of the NGS panel. Furthermore, by eliminating such sites in the design of a new NGS panel, it is possible to reduce errors related to false negatives in NGS panel analysis.

### Example 8. Analysis result of false positive variant and stringency cutoff value for NGS panel

As mentioned above, among the null alleles in which no variant was found in either the H mole DNA or the control genomic DNA (defined as "N-N pair alleles"), if variants were detected in the NGS panel analysis of the DNA mixture samples-where H mole DNA and control genomic DNA were mixed at different dilution ratios-these were classified as false positive variants. Subsequently, the detection frequency of false positive variants in each company's NGS panel was analyzed.

As a result, the NGS panels of companies AA and BB were found to have relatively fewer false positive variants compared to that of CC Company (FIG. 7). This is believed to be due to the VAF values representing the limits of detection for the NGS panels of companies AA and BB being relatively higher than the limit of detection VAF values of CC Company, indicating a decrease in sensitivity to detect variants. However, the NGS panel of CC Company was confirmed to have relatively fewer false positive variants compared to the NGS panels of companies AA and BB when the VAF was 0.1 or higher, except for chromosome 6 (FIG. 7).

For reference, two issues were identified in the CC Company's NGS panel analysis. First, a significant number of false positive variants were detected in a 10% DNA mixture sample (i.e., a DNA mixture containing 10% H mole DNA and 90% control genomic DNA (CH10)). If the experiment had been conducted under conditions similar to those of other samples, a comparable number of false positive variants should have been observed. However, since approximately ten times more false positive variants were found only in CH10, it was concluded that this represented an error in the experimental process using the CC Company's NGS device. Consequently, as shown in FIG. 7C, the false positive variants found solely in CH10 were excluded from the analysis. Second, the chromosome 6 alleles in the CC Company's NGS panel exhibited an issue where the VAF of false positive variants was significantly lower or higher than that of false positive variants at other chromosome sites, as confirmed in FIG. 7C. Therefore, alleles on chromosome 6 were excluded from the false positive variant detection analysis.

Subsequently, as mentioned above, the 95% stringency cutoff value or the 99% stringency cutoff value, defined as the VAF values that can eliminate 95% or 99% of false positive variants, was derived. To date, most researchers have identified only those variants above a certain VAF value as "true variants" to reduce false positive variants following NGS panel analysis. This VAF value is referred to as the "stringency cutoff value," but clear scientific standards for determining this value have not yet been established.

The present invention provides a clear method for deriving a VAF value that can eliminate 95% or 99% of false positive variants, referred to as the "stringency cutoff value." To validate this method, the distribution of VAF values for false positive variants in the N-N pair alleles of CC Company was analyzed. In this case, alleles corresponding to the two identified issues (i.e., 1. large-scale false positive variants in CH10 samples and 2. significant changes in VAF for false positive variants on chromosome 6) that were problematic in CC Company's NGS panel were excluded from the analysis before deriving the stringency cutoff value.

As a result, it was confirmed that the total number of false positive variants obtained from CC Company's NGS panel was 1,977. The VAF value needed to eliminate 95% of the false positive variants was 0.067, while the VAF value required to remove 99% of the false positive variants was 0.0875 (FIG. 8). Thus, to analyze CC Company's NGS panel and remove 95% of false positive variants, the derived stringency cutoff value is 0.067. Conversely, to achieve the removal of 99% of false positive variants, the derived stringency cutoff value is 0.087.

From the above results, it was confirmed that the present invention can validate the NGS panel by analyzing the frequency of false positive variants and provide information on an objective stringency cutoff value to eliminate false positive variants, thereby increasing the specificity of the NGS panel. This method effectively reduces errors related to the interpretation of NGS results associated with false positives occurring in the NGS panel.

### Example 9. Evaluation of errors contained in FASTQ files and errors of bioinformatics analysis in the NGS panel analysis results of each company

The errors in the results of each company's analysis of the NGS panels can be categorized into two types: 1) errors in the raw data generated using the NGS panel, specifically the FASTQ file itself, and 2) errors in the bioinformatics analysis process used to identify variants from the raw data of each company. To assess the contribution of these two types of errors, commercial software was employed to identify variants from the raw data, and Illumina's DRAGEN software was used for the bioinformatics analysis. Although this process requires bed files from each company, companies AA and CC did not provide these files, so only the exon-part variants of the genes provided by the two companies could be analyzed. In contrast, BB Company allowed for the analysis of all variants across the entire captured site using the bed file it provided.

The determination of false negative errors in the variant part was conducted as described above. To identify false positive errors, any cases where a variant or reference base that was not present in either H mole DNA or control genomic DNA was detected in all alleles-including N-N pair alleles, Ho-N pairs, He-N pairs, and Ho-He pairs-were classified as false positive errors. Subsequently, the detection frequency of false positive variants in each company's NGS panel was analyzed.

FIG. 9 presents the results of analyzing false negatives and false positives from the variant results provided by the bioinformatics methods of the three companies, as well as the results from analyzing false negatives and false positives obtained by examining the variants from the raw data (FASTQ files) of each company using DRAGEN software. The variant analysis was conducted using DRAGEN software under three conditions: default, solid, and liquid. The default condition is commonly used, while the solid and liquid conditions provide higher sensitivity than the default condition.

FIG. 9A illustrates the results of analyzing the false positive variants among the derived variants. The graph shows the false positive variant analysis results from each company's (In-house) variants as well as the results from variants derived using DRAGEN software under three conditions: default, solid, and liquid. The y-axis in FIG. 9A represents the arithmetic mean value of false positive variants found in multiple diluted samples. It can be observed that the results analyzed under default conditions of the DRAGEN software show fewer false positives compared to those analyzed under the solid or liquid conditions (with the number of false positives for companies BB and CC being 30 or fewer, as indicated by the blue line). Given that the default conditions for DRAGEN software analysis have lower sensitivity than the other two conditions, this finding aligns with the general observation that specificity tends to increase when sensitivity is reduced.

However, when analyzing variants using the in-house method of AA Company, the results indicated that there were few false positive variants, resulting in an average of only 0.5 false positives. In contrast, when analyzed using the default condition of DRAGEN software, a significant number of false positives were observed (FIG. 9A quadrilateral, with an average of 818 false positives). For this reason, as will be discussed later, it is suspected that numerous false positive errors occurred in the analysis of AA Company's NGS panel results, suggesting that the conditions were altered to increase specificity at the expense of sensitivity. Additionally, it can be seen that CC Company's in-house method generates a high number of false positives (indicated by the arrow in FIG. 9A, with an average of 1680), which appears to be related to CC Company's intentional decision to increase sensitivity for variant analysis, as will be elaborated upon later.

FIG. 9B presents the results of measuring sensitivity by analyzing false negatives from the variant detection outcomes of the NGS panels of companies AA, BB, and CC. The graph displays the results of analyzing false negative variants derived from each company's respective bioinformatics methods (In-house) as well as the results of analyzing the false negative variants derived from DRAGEN software's bioinformatics method under three conditions (default, solid, and liquid) for the detection rate of diluted variants (y-axis). Additionally, the percentage shown on the x-axis of FIG. 9B represents the percentage of samples with heterozygous variants. Therefore, the percentage of variants in the total DNA of each sample is half of the percentage of variant samples shown on the x-axis (for example, a sample marked as 5% on the x-axis in FIG. 9B indicates that the actual variant fraction is 2.5%). All results using the bioinformatics method with DRAGEN software in FIG. 9B are based on the default condition. To analyze the mutation detection rate using DRAGEN software in FIG. 9B, only variants present in the exons of the target genes provided by each company were analyzed for companies AA and CC, while variants present in all sites captured by BB Company's NGS panel were analyzed using the bed file. Additionally, all sensitivity analysis results in FIG. 9B are derived from He-N pair alleles.

Examining the sensitivity analysis results in FIG. 9B, the results obtained by AA Company using their bioinformatics method indicated that variants were hardly detected in samples containing 20% heterozygous variant DNA. This suggests that the variant measurement limit of AA Company's NGS panel is greater than 10%. Additionally, based on the results of analyzing the Ho-N pair alleles, the final analysis using AA Company's raw data and their bioinformatics method indicates that the variant measurement limit is approximately 20%. In contrast, when analyzing the variants obtained under the default condition of DRAGEN software, it was found that most of the variants in samples containing 10% heterozygous variant DNA were detected, indicating that the sensitivity variant measurement limit is around 5%. Therefore, it appears that the bioinformatics method employed by AA Company selected conditions with low sensitivity. This low sensitivity adjustment seems to be linked to the discovery of a substantial number of false positive variants (818) when analyzing the variants obtained using the default condition of DRAGEN software in FIG. 9A. It can be inferred that AA Company modified the analysis conditions to enhance specificity at the expense of sensitivity, resulting in a sensitivity threshold of approximately 20%.

In the case of CC Company, the sensitivity analysis results in FIG. 9B show that most of the diluted variants in the 2.5% heterozygous variant DNA sample were detected based on the analysis results using their bioinformatics conditions, indicating that the sensitivity is capable of detecting most 1.25% variants. However, when analyzing the variants obtained using the default condition of DRAGEN software, it appears that only about 50% of the diluted variants in the 2.5% heterozygous variant DNA sample are detected, while most of the diluted variants in the 5% heterozygous variant DNA sample are detected, suggesting that the limit of detection for variants is around 2.5%. Therefore, CC Company's bioinformatics method seems to demonstrate higher sensitivity compared to the DRAGEN software analysis. However, examining the specificity analysis results in FIG. 9A reveals that CC Company's in-house bioinformatics analysis conditions detected 1,680 false positive variants. This indicates that their bioinformatics analysis intentionally employed conditions that enhance sensitivity, which consequently results in a significant reduction in specificity.

In summary, it can be evaluated that AA Company encountered numerous errors in the process of generating raw data and appears to have adjusted their analysis conditions to sacrifice sensitivity in order to address the issue of a high number of false positive variants during the bioinformatics analysis process. BB Company reported a higher occurrence of false negative variants during the bioinformatics analysis compared to variant analysis conducted using DRAGEN software. Consequently, the sensitivity of the NGS panel analyzed by BB Company appears to be slightly lower than the sensitivity indicated by the analysis using DRAGEN software. Meanwhile, CC Company adjusted their analysis conditions to enhance sensitivity during the bioinformatics analysis process, which resulted in a significant number of false positives.

As described above, by analyzing the results of the present invention using commercial software, there is an advantage in that, during the NGS panel analysis process of a specific company, it is possible to separately identify: 1) errors in the process of generating raw data and 2) errors in the bioinformatics analysis process.

## Claims

1. A composition for validating a next generation sequencing (NGS) panel, comprising homozygote DNA and control genomic DNA.

2. The composition of claim 1, wherein the homozygote DNA includes DNA isolated from a hydatidiform mole cell line or a parthenogenetic cell line.

3. The composition of claim 1, wherein the control genomic DNA includes genomic DNA isolated from the blood of a normal individual.

4. The composition of claim 1, wherein the control genomic DNA includes genomic DNA isolated from the blood or cancer tissue of a patient.

5. The composition of claim 1, wherein the control genomic DNA includes circulating tumor DNA (ctDNA).

6. The composition of claim 1, wherein the control genomic DNA includes synthetic DNA or cloned DNA.

7. The composition of claim 1, wherein the dilution ratios of the homozygote DNA and the control genomic DNA are selected from 1:99 to 99:1.

8. The composition of claim 1, wherein the dilution ratios of the homozygote DNA to the control genomic DNA are selected from the group consisting of 1:9999, 2.5:9997.5, 5:9995, 1:999, 2.5:997.5, 5:995, 1:99, 2.5:97.5, 5:95, 10:90, 20:80, 50:50, 80:20, 90:10, 95:5, 97.5:2.5, 99:1, 995:5, 997.5:2.5, 999:1, 9995:5, 9997.5:2.5 and 9999:1.

9. A kit for validating a next generation sequencing (NGS) panel, comprising the composition for validating an NGS panel according to any one of claims 1 to 8.

10. A method for validating a next generation sequencing (NGS) panel through the analysis of false negative variants, the method comprising:
(a) performing NGS using a NGS panel on DNA mixture samples, wherein the DNA mixture samples consist of homozygote DNA and control genomic DNA at various dilution ratios;
(b) selecting Ho-N pair alleles (Homozygote-Null pair alleles) or He-N pair alleles (Heterozygote-Null pair allele) from the NGS results; and
(c) analyzing the frequency of false negative variants in the DNA mixture samples based on the selected Ho-N pair or He-N pair alleles, wherein the DNA mixture samples are composed of homozygote DNA and control genomic DNA at various dilution ratios.

11. A method for validating a next generation sequencing (NGS) panel through the analysis of limit of detection, the method comprising:
(a) performing NGS using a NGS panel on DNA mixture samples, wherein the DNA mixture samples consist of homozygote DNA and control genomic DNA at various dilution ratios;
(b) selecting Ho-N pair alleles (Homozygote-Null pair alleles) or He-N pair alleles (Heterozygote-Null pair alleles) from the NGS results;
(c) analyzing the detection rate of diluted variants in DNA mixture samples based on the selected Ho-N pair alleles or the He-N pair alleles, wherein the DNA mixture samples are composed of homozygote DNA and control genomic DNA at various dilution ratios, and wherein the detection rate of diluted variants is expressed as a percentage of the number of diluted variants found among the Ho-N pair alleles or He-N pair alleles; and
(d) analyzing limit of detection.

12. The method of claim 11, wherein the NGS panel is configured to detect variants selected from the group consisting of single nucleotide variants (SNVs), insertions/deletions (Indels), and chromosomal amplifications/deletions.

13. The method of claim 11, wherein the limit of detection in the analysis is expressed as the lowest variant allelic fraction (VAF) value at which 90% or 95% of the diluted variants are detected in the DNA mixture samples, wherein the DNA mixture samples consist of homozygote DNA and control genomic DNA at various dilution ratios.

14. A method for validating a next generation sequencing (NGS) panel through the analysis of false positive variants, the method comprising:
(a) performing NGS using a NGS panel on DNA mixture samples, wherein the DNA mixture samples consist of homozygote DNA and control genomic DNA at various dilution ratios;
(b) selecting N-N pair alleles (Null-Null pair alleles) from the NGS result, wherein the N-N pair alleles do not include any variants in either the homozygote DNA or the control genomic DNA; and
(c) analyzing the frequency of false positive variants in DNA mixture samples based on the selected N-N pair alleles, wherein the DNA mixture samples are composed of homozygote DNA and control genomic DNA at various dilution ratios.

15. The method of claim 14, wherein the NGS panel is configured to detect variants selected from the group consisting of single nucleotide variants (SNVs), insertions/deletions (Indels), and chromosomal amplifications/deletions.

16. A method for providing information for increasing the specificity of a next generation sequencing (NGS) panel, the method comprising:
(a) performing NGS using a NGS panel on DNA mixture samples, wherein the DNA mixture samples consist of homozygote DNA and control genomic DNA at various dilution ratios;
(b) selecting N-N pair alleles (Null-Null pair alleles) homozygote DNA from the result of performing the NGS, wherein the N-N pair alleles do not include any variants in either the homozygote DNA or the control genomic DNA;
(c) analyzing the frequency of false positive variants in DNA mixture samples based on the selected N-N pair alleles, wherein the DNA mixture samples are composed of homozygote DNA and control genomic DNA at various dilution ratios; and
(d) deriving a stringency cutoff value by analyzing the variant allelic fraction (VAF), which eliminates the false positive variants.

17. The method of claim 16, wherein the stringency cutoff value is a 90% at which 90% of false positive variants are removed; a 95% at which 95% of false positive variants are removed; or a 99% at which 99% of false positive variants are removed.

18. The method of claim 16, wherein the method provides information on a stringency cutoff value to enhance the specificity of a next generation sequencing (NGS) panel, wherein the stringency cutoff removes false positive variants.

19. A method for evaluating errors in the process of generating raw data or errors in the bioinformatics analysis during the next generation sequencing (NGS) panel analysis process of a company, the method comprising:
(a) collecting false negative and false positive variants using the bioinformatics analysis results of the company based on the company's raw data; and
(b) comparing and analyzing false negative and false positive variants with commercial bioinformatics software using the company's raw data.
